# FASCICULE DE BREVET EUROPEEN

(11) **EP 1 338 591 B1**
(45) Date de publication et mention de la délivrance du brevet: **26.10.2005**
(21) Numéro de dépôt: 03290487.2
(22) Date de dépôt: 28.02.2003
(51) Int. Cl.: C07D 209/42

(54) **Nouveau procédé de synthèse de l'acide (2S, 3aS, 7aS)-perhydroindole-2-carboxylique et de ses esters, et application à la synthèse du perindopril**
Verfahren zur Synthese des (2S,3aS,7aS)-perhydroindol-2-carbonsäures und seiner Estern, Verwendung in der Synthese von Perindopril
Method for synthesis of (2S,3aS,7aS)-perhydroindole-2- carboxylic acid and esters thereof, and use in the synthesis of perindopril

(43) Date de publication de la demande: 27.08.2003
(73) Titulaire: Les Laboratoires Servier, 92415 Courbevoie Cedex (FR)
(72) Inventeur: Dubuffet, Thierry, 76210 Bolbec (FR); Langlois, Pascal, 76210 Saint Jean de la Neuville (FR)

(56) Documents cités:
- EP-A- 0 937 714
- WO-A-01/58868
- WO-A-03/016336
- VINCENT M ET AL: "Stereoselective Synthesis of a New Perhydroindole Derivative of Chiral Iminodiacid, a Potent Inhibitor of Agiotensin Converting Enzyme" TETRAHEDRON, ELSEVIER SCIENCE PUBLISHERS, AMSTERDAM, NL, vol. 23, no. 16, 1982, pages 1677-1680, XP002155080 ISSN: 0040-4020

## Description

La présente invention concerne un procédé de synthèse de l'acide (2S, 3aS, 7aS)-perhydroindole-2-carboxylique et de ses esters, et leur application à la synthèse du perindopril et de ses sels pharmaceutiquement acceptables.

Plus spécifiquement, la présente invention concerne un nouveau procédé de synthèse des dérivés de formule (I) : 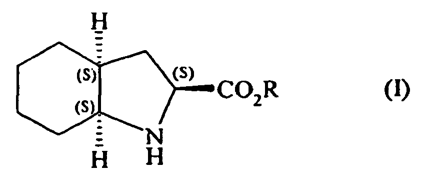 dans laquelle R représente un atome d'hydrogène ou un groupement protecteur de la fonction acide,
ainsi que leurs sels d'addition à un acide ou à une base minéral(e) ou organique.

Parmi les groupements protecteurs de la fonction acide, on peut citer à titre non limitatif les groupements benzyle et alkyle (C₁-C₆) linéaire ou ramifié.

Les composés de formule (I) obtenus selon le procédé de l'invention sont utiles dans la synthèse du perindopril de formule (II) : 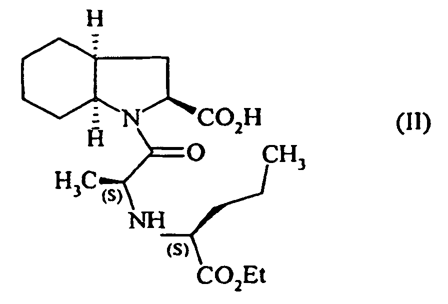 ainsi que dans celle de ses sels pharmaceutiquement acceptables.

Le perindopril, ainsi que ses sels, possèdent des propriétés pharmacologiques intéressantes.

Leur principale propriété est d'inhiber l'enzyme de conversion de l'angiotensine I (ou kininase II), ce qui permet d'une part d'empêcher la transformation du décapeptide angiotensine 1 en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir accéder à l'intermédiaire de formule (I) avec un procédé de synthèse performant, permettant l'obtention sélective du diastéréoisomère (S,S,S) avec un bon rendement et une excellente pureté, à partir de matières premières bon marché.

Quelques méthodes de préparation des composés de formule (I) sont déjà connues.

Ainsi, le brevet EP 0 037 231 utilise comme matière première l'acide indole 2-carboxylique, qui est soumis à une hydrogénation catalytique sur rhodium pour donner un mélange des deux isomères cis endo de configurations respectives (2S, 3aS, 7aS) et (2R, 3aR, 7aR). Ce mélange est ensuite séparé de façon particulièrement laborieuse : synthèse du dérivé N-benzoylé, cristallisation fractionnée du sel du diastéréoisomère avec la (S)-α-phényl-éthylamine, libération des deux dérivés (S, S, S) et (R, R, R) N-benzoylés, puis élimination du groupement benzoyle, suivie d'un passage sur colonne échangeuse d'ions et d'une recristallisation.

Le brevet EP 0 115 345, pour cette même séparation, utilise plusieurs étapes nécessitant l'estérification de la fonction acide carboxylique par l'alcool benzylique, la salification de l'amino ester par la N-benzyloxycarbonyl-(S)-phénylalanine, la séparation par cristallisation fractionnée de l'isomère (S, S, S), la libération de la fonction aminée optionnellement suivie de la libération du groupement acide carboxylique.

Les brevets EP 0 308 339 et EP 0 308 341 utilisent également comme matière première l'acide indole 2-carboxylique, qui est dans un premier temps réduit en acide indoline 2-carboxylique, pour donner un mélange d'acide indoline carboxylique 2R et 2S, lesquels sont ensuite séparés par cristallisation fractionnée. L'isomère 2S est ensuite soumis à hydrogénation catalytique pour conduire au composé de formule (I).

La demanderesse a présentement mis au point un nouveau procédé de synthèse des dérivés de formule (I), à partir d'une matière première particulièrement bon marché, et qui permet l'obtention sélective du diastéréoisomère (S, S, S) avec un bon rendement et une excellente pureté.

Plus spécifiquement, la présente invention concerne un procédé de synthèse des composés de formule (I), caractérisé en ce que l'on fait réagir la 1-(1-cyclohexén-1-yl)-pyrrolidine de formule (III) : 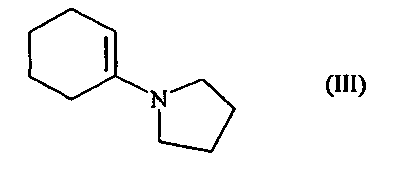 avec le composé de formule (IV): 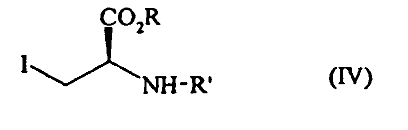 dans laquelle R est tel que défini dans la formule (I), et R' représente un groupement protecteur de la fonction aminé qui est différent de R,
pour conduire au composé de formule (V) : 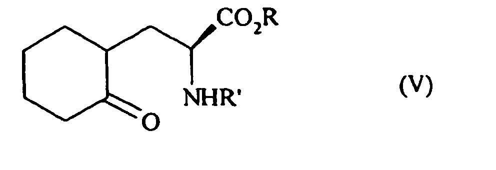 dans laquelle R et R' sont tels que définis précédemment,
dont on déprotège la fonction amine avant d'en effectuer la cyclisation suivie de la déshydratation, pour conduire au composé de formule (VI) : 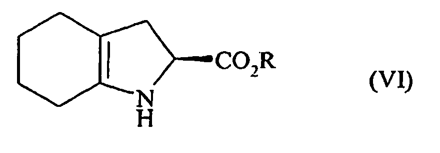 dans laquelle R est tel que défini précédemment,
que l'on soumet à une hydrogénation catalytique, en présence d'un catalyseur tel que le platine, le palladium, le rhodium ou le nickel,
sous une pression comprise entre 1 et 30 bars, de préférence entre 1 et 10 bars, pour conduire, après éventuelle déprotection ou reprotection de la fonction acide, au composé de formule (1).

Le composé de formule (I) ainsi obtenu a une très bonne pureté chimique et énantiomérique, ce qui rend son emploi particulièrement avantageux dans la synthèse du perindopril de formule (II).

A titre d'illustration, le couplage du composé de formule (I) obtenu selon le procédé de l'invention avec le composé de formule (VII) : 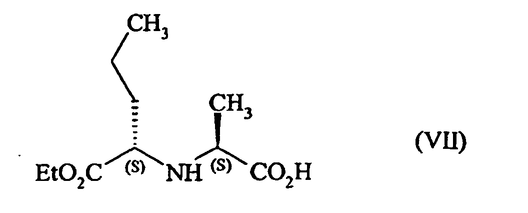 permet d'obtenir le perindopril de formule (II) avec une pureté et un rendement très satisfaisants.

Les exemples ci-dessous illustrent l'invention mais ne la limitent en aucune façon.

### EXEMPLE 1 : Acide (2S, 3aS, 7aS)-perhydroindole-2-carboxylique

### Stade A : (2S)-2-[(Tert-butyloxycarbonyl)-amino]-3-(2-oxocyclohexyl)-propanoate de benzyle

Dans un réacteur équipé d'une colonne à reflux, placer 200 g de 1-(1-cyclohexén-1-yl)-pyrrolidine, 535 g de (2S)-2-[(tert-butyloxycarbonyl)-amino]-3-iodopropanoate de benzyle et 1,5 1 d'acétonitrile.
Amener au reflux pendant 1 h, puis ramener le mélange à température ambiante. Après évaporation du solvant, ajouter 2 1 d'eau, puis de l'acide acétique. Extraire par l'acétate d'éthyle et évaporer à sec. Le (2S)-2-[(tert-butyloxycarbonyl)-amino]-3-(2-oxocyclohexyl)-propanoate de benzyle est ainsi obtenu avec un rendement de 80 %.

### Stade B : (2S)-2-Amino-3-(2-oxocyclohexyl)-propanoate de benzyle

Dans un réacteur, placer 200 g du composé obtenu dans le stade précédent, 1,5 1 de dichlorométhane et 60 g d'acide trifluoroacétique. Après 1 h30 d'agitation à température ambiante, ajouter 2 1 d'une solution saturée d'hydrogénocarbonate de sodium. Extraire par le dichlorométhane et évaporer à sec.
Le (2S)-2-amino-3-(2-oxocyclohexyl)-propanoate de benzyle est ainsi obtenu avec un rendement de 90 %.

### Stade C : (2S)-2,3,4,5,6,7-Hexahydro-1H-indole-2-carboxylate de benzyle

Dans un réacteur, chauffer à reflux 200 g du composé obtenu dans le stade précédent, 13,8 g d'acide p-toluènesulfonique et 1 1 de toluène en éliminant l'eau formée par entraînement azéotropique. Lorsqu'il ne décante plus d'eau, évaporer le toluène.
Le (2S)-2,3,4,5,6,7-hexahydro-1*H*-indole-2-carboxylate de benzyle est ainsi obtenu avec un rendement sur brut de 97 %.

### Stade D : Acide (2S, 3aS, 7aS)-perhydroindole-2-carboxylique

Dans un hydrogénateur, placer 200 g du composé obtenu dans le stade précédent, en solution dans l'acide acétique, puis 5 g de Pt/C à 10 %. Hydrogéner sous pression de 5 bars à température ambiante, jusqu'à absorption de la quantité théorique d'hydrogène.
Eliminer le catalyseur par filtration, puis refroidir entre 0 et 5°C et récolter le solide obtenu par filtration. Laver le gâteau et le sécher jusqu'à poids constant.
L' acide (2S, 3aS, 7aS)-perhydroindole-2-carboxylique est ainsi obtenu avec un rendement de 87 % et une pureté énantiomérique de 99 %.

### EXEMPLE 2 : (2S, 3aS, 7aS)-Perhydroindole-2-carboxylate de benzyle, paratoluènesulfonate

Dans un réacteur, chauffer à reflux 200 g du composé de l'exemple 1, 800 g d'acide paratoluènesulfonique, 227 g d'alcool benzylique et 700 ml de toluène en éliminant l'eau formée à l'aide d'un décanteur en continu. Lorsqu'il ne décante plus d'eau, refroidir, essorer le précipité fomié et sécher.
Le paratoluènesulfonate du (2S, 3aS, 7aS)-perhydroindole-2-carboxylate de benzyle est ainsi obtenu avec un rendement de 91 % et une pureté énantiomérique de 99 %.

## Revendications

1. Procédé de synthèse des composés de formule (I) : 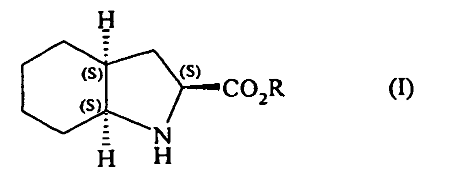 dans laquelle R représente un atome d'hydrogène ou un groupement protecteur de la fonction acide,
**caractérisé en ce que** l'on fait réagir la 1-(1-cyclohexén-1-yl)-pyrrolidine de formule (III) : 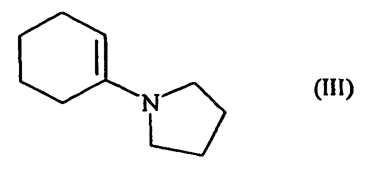 avec le composé de formule (IV) : 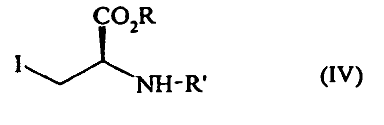 dans laquelle R est tel que défini dans la formule (I), et R' représente un groupement protecteur de la fonction amine qui est différent de R,
pour conduire au composé de formule (V) : 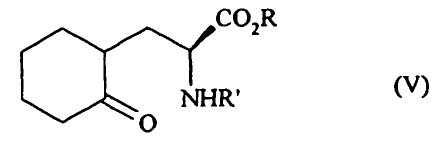 dans laquelle R et R' sont tels que définis précédemment,
dont on déprotège la fonction amine avant d'en effectuer la cyclisation suivie de la déshydratation, pour conduire au composé de formule (VI) : 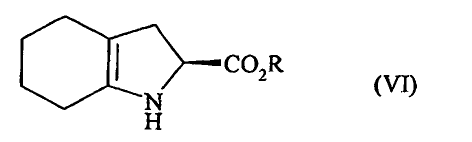 dans laquelle R est tel que défini précédemment,
que l'on soumet à une hydrogénation catalytique, en présence d'un catalyseur tel que le platine, le palladium, le rhodium ou le nickel,
sous une pression comprise entre 1 et 30 bars, pour conduire, après éventuelle déprotection ou reprotection de la fonction acide, au composé de formule (I).

2. Procédé de synthèse selon la revendication 1, **caractérisé en ce que** la pression d'hydrogène de la réaction d'hydrogénation est comprise entre 1 et 10 bars.

3. Procédé de synthèse selon la revendication 1, permettant l'obtention du dérivé de formule (I) dans laquelle R représente un atome d'hydrogène.

4. Procédé de synthèse selon la revendication 1, permettant l'obtention du dérivé de formule (I) dans laquelle R représente le groupement benzyle.

5. Procédé de synthèse du perindopril ou de ses sels phannaceudquement acceptables à partir d'un composé de formule (I), **caractérisé en ce que** ledit composé de formule (I) est obtenu selon le procédé de la revendication 1.

## Patentansprüche

1. Verfahren zur Synthese der Verbindungen der Formel (I): 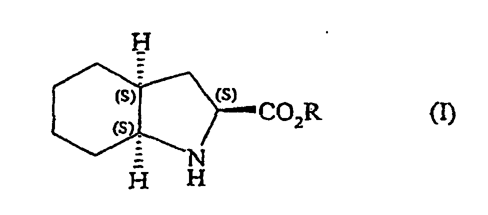 in der R ein Wasserstoffatom oder eine Schutzgruppe für die Säurefunktion bedeutet,
**dadurch gekennzeichnet, daß** man 1-(1-Cyclohexen-1-yl)-pyrrolidin der Formel (III): 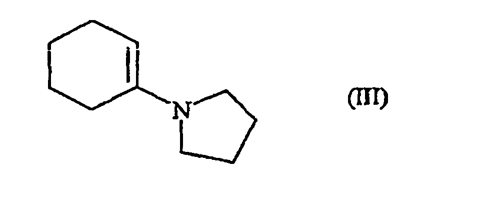 mit der Verbindung der Formel (IV): 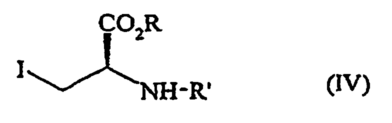 in der R die bezüglich der Formel (I) angegebenen Bedeutungen besitzt und R' eine von R verschiedene Schutzgruppe für die Aminfunktion bedeutet, umsetzt zur Bildung der Verbindung der Formel (V): 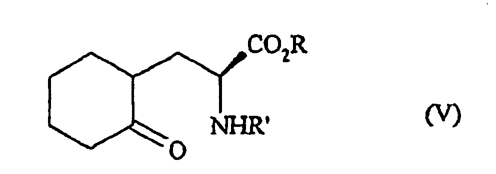 in der R und R' die oben angegebenen Bedeutungen besitzen,
von welcher man die Schutzgruppe der Aminfunktion abspaltet bevor man die Cyclisierung gefolgt von einer Dehydratisierung durchführt zur Bildung der Verbindung der Formel (VI): 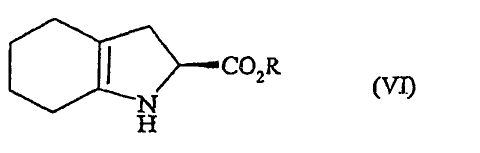 in der R die oben angegebenen Bedeutungen besitzt,
welche man einer katalytischen Hydrierung in Gegenwart eines Katalysators, wie Platin, Palladium, Rhodium, oder Nickel, unter einem Druck zwischen 1 und 30 bar unterwirft, so daß man nach der eventuellen Abspaltung der Schutzgruppe oder dem erneuten Schutz der Säurefunktion die Verbindung der Formel (I) erhält.

2. Syntheseverfahren nach Anspruch 1, **dadurch gekennzeichnet, daß** der Wasserstoffdruck der Hydrierungsreaktion zwischen 1 und 10 bar liegt.

3. Syntheseverfahren nach Anspruch 1 für die Herstellung des Derivats der Formel (I), in der R ein Wasserstoffatom bedeutet.

4. Syntheseverfahren nach Anspruch 1 für die Herstellung des Derivats der Formel (I), in der R die Benzylgruppe darstellt.

5. Verfahren zur Synthese von Perindopril oder von seinen pharmazeutisch annehmbaren Salzen, ausgehend von einer Verbindung der Formel (I), **dadurch gekennzeichnet, daß** die Verbindung (I) nach dem Verfahren des Anspruchs 1 hergestellt wird.

## Claims

1. Process for the synthesis of compounds of formula (I) : 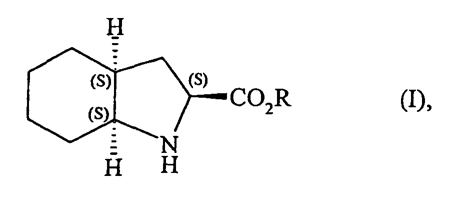 wherein R represents a hydrogen atom or a protecting group for the acid function,
**characterised in that** 1-(1-cyclohexen-1-yl)-pyrrolidine of formula (III) : 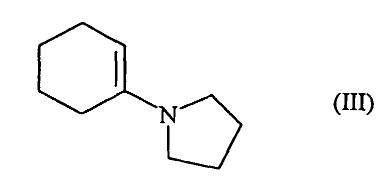 is reacted with the compound of formula (IV) : 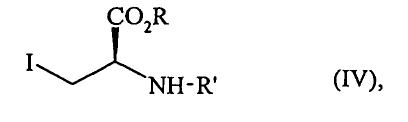 wherein R is as defined for formula (I) and R' represents a protecting group for the amine function that is different from R,
to yield the compound of formula (V) : 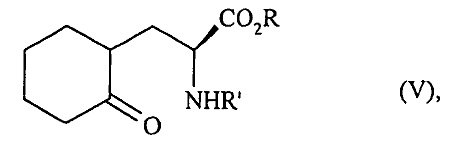 wherein R and R' are as defined hereinbefore,
the amine function of which is deprotected before carrying out cyclisation followed by dehydration to yield the compound of formula (VI) : 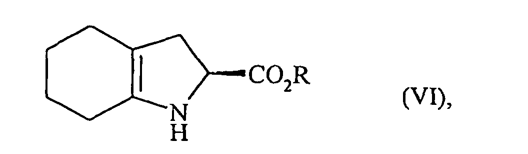 wherein R is as defined hereinbefore,
which is subjected to catalytic hydrogenation, in the presence of a catalyst such as platinum, palladium, rhodium or nickel,
under a pressure of from 1 to 30 bars, to yield, after optional deprotection or reprotection of the acid function, the compound of formula (I).

2. Synthesis process according to claim 1, **characterised in that** the hydrogen pressure in the hydrogenation reaction is from 1 to 10 bars.

3. Synthesis process according to claim 1, allowing the compound of formula (I) wherein R represents a hydrogen atom to be obtained.

4. Synthesis process according to claim 1, allowing the compound of formula (I) wherein R represents a benzyl group to be obtained.

5. Process for the synthesis of perindopril or pharmaceutically acceptable salts thereof starting from a compound of formula (I), **characterised in that** the said compound of formula (I) is obtained according to the process of claim 1.
